(19) Europäisches Patentamt
European Patent Office
Office européen des brevets

(11) Publication number: **0 592 437 B1**

# (12) EUROPEAN PATENT SPECIFICATION

(45) Date of publication of patent specification: **27.12.95**

(51) Int. Cl.[6]: **C10M 151/04**, C10L 1/24, C08G 75/14, C07C 37/14

(21) Application number: **91920180.6**

(22) Date of filing: **09.10.91**

(86) International application number: **PCT/US91/07558**

(87) International publication number: **WO 92/07052 (30.04.92 92/10)**

## (54) PROCESS FOR PREPARING ALKYL PHENOL-SULFUR CONDENSATE LUBRICATING OIL ADDITIVES (PT-790)

(30) Priority: **10.10.90 US 595229**

(43) Date of publication of application: **20.04.94 Bulletin 94/16**

(45) Publication of the grant of the patent: **27.12.95 Bulletin 95/52**

(84) Designated Contracting States: **BE DE FR GB IT NL**

(56) References cited:
**EP-A- 0 249 415**
**EP-A- 0 311 450**
**WO-A-88/03133**
**DE-C- 1 199 277**
**FR-A- 1 498 053**
**FR-A- 2 370 020**
**US-A- 2 789 143**

(73) Proprietor: **EXXON CHEMICAL PATENTS INC.**
**1900 East Linden Avenue**
**Linden,**
**New Jersey 07036-0710 (US)**

(72) Inventor: **MARTELLA, David, John**
**11F Brookline Court**
**Princeton, NJ 08540 (US)**
Inventor: **JARUZELSKI, John, Jan**
**474 Channing Avenue**
**Westfield, NJ 07090 (US)**
Inventor: **CHEN, Frank, Joung-Yei**
**28 Ellison Avenue**
**Edison, NJ 08820 (US)**

(74) Representative: **Northover, Robert Frank et al**
**Exxon Chemical Limited**
**Exxon Chemical Technology Centre**
**PO Box 1**
**Abingdon**
**Oxfordshire, OX13 6BB (GB)**

EP 0 592 437 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to multi-functional additives for improving both the low temperature flow properties and the oxidation stability of hydrocarbon oils. More particularly, the present invention relates to an improved process for preparing additives for improving the low temperature flow properties and oxidation stability of various hydrocarbon oil compositions.

A large variety of additives for improving various properties in hydrocarbon oil compositions are well known, and in fact a large number of these compositions are being used on a commercial level. The various additives are used for a variety of purposes, some of which relate to improving the low temperature (i.e. less than about 30°F) flow properties of various types of hydrocarbon oils, including both lubricating oil fractions and other oil fractions including heating oils, diesel oils, middle distillates, and the like, and others of which relate to improving the oxidation stability of these various types of hydrocarbon oils. These flow improvers generally modify the wax crystals in both lubricating oils and other hydrocarbon fractions and crudes so as to impart low temperature handling, pumpability, and/or vehicle operability thereto. These parameters are generally measured by a variety of tests, including pour point, cloud point, mini-rotary viscometry (MRV) and others. Those other additives are used primarily for imparting anti-oxidant properties to these hydrocarbon fractions, including lubricating oil fractions.

Cloud point (ASTM D 2500) is the temperature at which wax crystals first appear as a haze in a hydrocarbon oil upon cooling. Such wax crystals typically have the highest molecular weight of the waxes in the hydrocarbon oil and therefore the lowest solubility. The cloud point of a hydrocarbon oil reflects the temperature at which problems in filtering the oil are encountered. However, the cloud point of a lubricating oil (as opposed to a fuel oil) is of less significance than is its pour point because the filters typically encountered by a lubricating oil (e.g., combustion engine oil filters) have a relatively large pore size, and filter plugging is therefore less of a problem in these environments.

Pour point is the lowest temperature at which a hydrocarbon oil will pour or flow when chilled, without disturbance, under specified conditions. Pour point problems arise through the formation of solid or semi-solid waxy particles in a hydrocarbon oil composition under chilled conditions. Thus, as the temperature of the oil is decreased, the distribution of such oil by pumping or siphoning is rendered difficult or impossible when the temperature of this oil is around or below the pour point of the oil. Consequently, when the flow of oil cannot be maintained, equipment can fail to operate.

It has therefore been necessary to develop various additives for the purpose of influencing the cold temperature flow properties of hydrocarbon oils.

The general term "lubricating oil flow improver" (LOFI) covers all those additives which modify the size, number, and growth of wax crystals in lube oils in such a way as to impart improved low temperature handling, pumpability, and/or vehicle operability as measured by such tests as pour point, cloud point, and mini rotary viscometry (MRV). The majority of lubricating oil flow improvers are polymers or contain polymers. These polymers are generally of two types, either backbone or sidechain.

The backbone variety, such as the ethylene-vinyl acetates (EVA), have various lengths of methylene segments randomly distributed in the backbone of the polymer, which associate or cocrystallize with the wax crystals inhibiting further crystal growth due to branches and non-crystallizable segments in the polymer.

The sidechain-type polymers, which are the predominant variety used as LOFIs, have methylene segments as the side chains, preferably as straight side chains. These polymers work similarly to the backbone type except the side chains have been found more effective in treating isoparaffins as well as n-paraffins found in lube oils. More specifically, LOFIs are typically derived from unsaturated carboxylic acids or anhydrides which are esterified to provide pendent ester groups derived from a mixture of alcohols. Most current commercial additives of this type thus require the use of relatively expensive alcohols for their production. Representative examples of this type of side chain LOFI include dialkyl fumarate/vinyl acetate copolymers and esterified styrene/maleic anhydride copolymers.

It would be extremely advantageous if additives could be developed which rely on less expensive olefins rather than alcohols in the synthesis of low temperature flow improvers without sacrificing the properties of conventional alcohol-based LOFIs. Several commercially unsuccessful attempts have been made in the past using alkylated phenol formaldehyde condensates.

Irrespective of the large number of additive compositions which have previously been proposed and utilized for altering both the flow properties and the oxidative stability of hydrocarbon oils and lubricating oil compositions, the search has continued for additional additive compositions which can improve both the flow characteristics and the oxidative stability of these various hydrocarbon compositions, and which can also be easily produced on an economical basis.

US-A-4976882, equivalent to EP-A-311450, describes sulfur bridged alkyl phenol condensates and their preparation from alkyl phenols.

The present invention, provides a method of producing such sulfur bridged alkyl phenol condensates capable of improving the low temperature flow properties and oxidation stability of hydrocarbon oils wherein the condensation reaction product is prepared by a method which comprises a method for producing a polymeric additive suitable for improving the low temperature flow properties and oxidative stability of hydrocarbon oil which comprises (1) preparing an alkylated phenol by the reaction in a dipolar aprotic cosolvent of (a) phenol and (b) linear alpha-olefin having (i) from 6 to 50 carbon atoms, (ii) an average carbon number of from 12 to 26; and (iii) not more than 10 mole % containing less than 12 carbon atoms and not more than 10 mole % containing more than 26 carbon atoms to form an alkylated phenol comprising at least 80 mole % difunctional alkylated phenol and having greater than 35 mole % of the alkyl groups derived from the linear alpha-olefin as alpha methyl-substituted linear alkyl groups; and (2) condensing said alkylated phenol of step (1) with a sulfurizing agent so as to produce a sulfur bridged condensate having a number average molecular weight of at least 3,000 (as measured by VPO) and molecular weight distribution of at least about 1.5, the bridge comprising a group of the formula $-(S)_x-$ where x is a number between 1 and 8.

The additive compositions of the present invention are prepared by the alkylation of phenol conducted in the presence of at least one dipolar aprotic cosolvent to minimize the amount of rearrangement and to import essential linearity to the alkyl group of the alkylate, followed by condensation with a sulfurizing agent so as to produce polymers having certain specified molecular weights. More particularly, the use of the specific linear alpha-olefins results in superior lubricating oil and fuel oil flow improvers relative to the alkyl phenol-sulfur condensates in the prior art, and which also exhibit significant anti-oxidant properties. As will be demonstrated, these particular polymers are particularly and unexpectedly superior in terms of their ability to co-crystalize with the wax crystals in these hydrocarbon oils which again have multi-functional characteristics.

Alkylation of the phenol is initially conducted with a linear alpha-olefin or blend of linear alpha-olefins which are terminal olefins, as contrasted to internal olefins. In this manner, it is possible to produce final polymers in which the alkyl group attached to the benzene ring is essentially linear. By "essentially linear" is meant greater than 35, preferably at least 40, and most preferably at least 50 mole percent of the alkyl groups derived from the olefin alkylating agent (exclusive of the alkyl groups of any optional tri-or tetrafunctional component described hereinafter for molecular weight enhancement) and attached to the aromatic ring of the phenol group in the alkylated product is linear, except for a methyl group pendant from the carbon attached to that aromatic ring. More specifically, since terminal alpha-olefins are employed for the alkylation of phenol in accordance herewith, the terminal olefins will attach to the aromatic ring at the beta carbon thereof, thereby leaving the alpha carbon as a methyl group pendant from the beta carbon of the original olefin. Thus, expressed differently, "essentially linear" means greater than 35 mole percent of the alkyl groups of the alkylated phenol are alpha methyl substituted linear alkyl. The primary alkyl phenol product desired from this alkylation step (after rearrangement as discussed hereinafter) will be linear to at least that extent.

The initial alkylation step itself is an exothermic reaction of phenol with these particular linear terminal alpha-olefins. This reaction can thus be shown as follows:

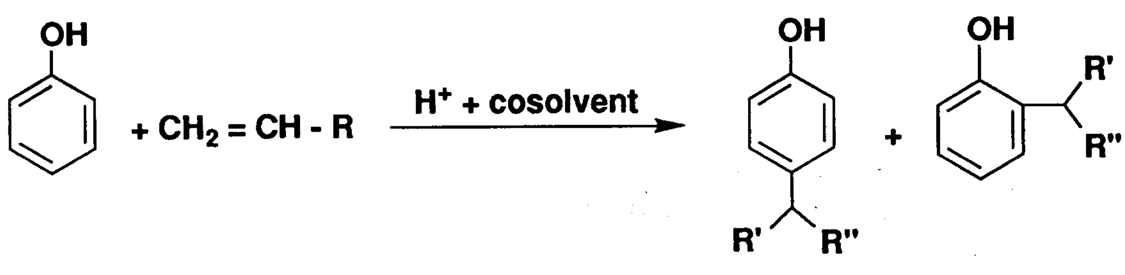


$\Delta H \approx$ -12K cal,

and in which R is linear alkyl, and R' and R" are linear alkyl groups derived in whole or in part from R. This exothermic reaction is thus a simple cationic reaction resulting in a somewhat complex product. In the ideal reaction the olefin forms a carbonium ion species as a result of the presence of acidic conditions and

temperatures. This cation can then readily react with phenol at either the ortho or para positions. Without rearrangement, the carbonium ion species will attach to the aromatic ring at the beta carbon of the olefin, and R' will thus constitute a pendant methyl group derived from the alpha carbon of the original olefin, with R'' constituting the remainder of the linear alkyl chain originally defined by R. In reality, however, many side reactions are possible. Thus, the cation can revert back to the olefin or rearrange further down the linear chain, thereby producing attachment to the aromatic ring at a more internal carbon atom, and causing the length of R' to increase, and R'' to decrease in length correspondingly. It has been found that if these rearrangements are too extensive, they will lead to the production of inferior products which would not suitably interact with the wax crystals of the lubricating oil or fuel oil to which they are eventually added.

It is therefore critical to the present invention to minimize these rearrangements and to maximize the attachment of the alkyl groups at the 2-position (i.e., beta carbon of the original linear olefin). The instant invention presents a novel method of producing alkyl phenols having essentially linear alkyl substituted groups. The present invention describes a me!hod to produce alkyl phenols with less rearrangement, and thereby an alkyl phenol-sulfur condensate with greater pour point depressancy. The method involves conducting the alkylation of phenol in the presence of at least one dipolar aprotic cosolvent. The cosolvent of the invention desirably has a dielectric constant of greater than 10 and preferably greater than 20 and desirably from 20 to 50. Typical examples of suitable cosolvents are 1,2-dichloroethane (e = 10.4), hexamethylphosphoramide (e = 21), N-methyl-pyrrolidone (e = 32), nitrobenzene (e = 35), nitromethane (e = 36), N-N-dimethylformamide (e = 37), acetonitrile (e = 36), sulfolane (e = 44) and dimethyl sulfoxide (e = 47). The use of these dipolar aprotic cosolvents in the alkylation reaction significantly minimizes the amount of rearrangement and maximizes the attachment of the alkyl groups at the 2-position which thereby increases the pour point depressancy of the resulting alkyl phenol-sulfur condensates. The amount of solvent used is not critical and it would be within the knowledge of one skilled in the art to determine suitable amount without undue experimentation. Generally, amounts of above 50 wt. % of the reactant is considered suitable. Amounts, however, greatly in excess of that stated above would not be considered detrimental, but may present problems with removal and would not be cost effective for the process. The minimum amount would be that necessary to produce the desired product.

Other methods for minimizing such rearrangement are disclosed in US-A-4976882 and 5039437, which are preferably used in combination with the instant invention. One method, for instance, comprises carrying out the alkylation process at lower reaction temperature as opposed to elevated reaction temperatures. Therefore, the alkylation process itself can generally be conducted at temperatures of at or below 100 °C, preferably at or below 90 °C, e.g., typically between 50 and 100 °C, and preferably between 50 and 90 °C, to minimize rearrangement.

It has also been observed in the applications referenced above, that rearrangement is more likely to occur at the para position than the ortho position. This is probably a result of a steric factor which permits greater accommodation of rearrangement at the para position to the hydroxyl group. Accordingly, the definition of "essentially linear" accounts for, and expresses the permissible limits of, the above-discussed autogenous rearrangement in forming the alkylate product. In short, "essentially linear" expresses the maximum degree of acceptable branching in the alkylate product which can be tolerated when starting with linear alpha-olefins. The degree of rearrangement is typically determined by $^1$H-NMR and/or by high pressure liquid chromatography.

Another critical aspect in the preparation was found to be the carbon number and carbon number distribution of olefins employed for alkylation.

The particular linear alpha-olefins used in connection with the alkylation step of the present invention are, as indicated above, crucial to the manufacture of the proper additives for use herein. In particular, these linear alpha-olefins have the formula $CH_2$ = CH - R, in which R is straight chain alkyl having between about 4 and about 48 carbon atoms, and in which the specific alpha-olefin or mixture of alpha-olefins used for this alkylation has an average carbon number (on a molar basis for mixtures of olefins) of from 12 to 26 (e.g. 14 to 24), preferably from 16 to 22 (e.g., 17 to 21, or 16 to 19), and most preferably from 18 to 20.

Moreover, the olefin mixture as was disclosed, should not contain more than about 10 mole percent, and preferably does not contain more than about 5 mole percent, and most preferably not more than about 2 mole percent of alpha-olefins having independently: (a) less than about 12, preferably not less than about 14, and most preferably not less than about 16 carbon atoms; and (b) not more than about 26, preferably not more than about 24, and most preferably not more than about 22 carbon atoms. These proportional requirements are thereby incorporated into, and embodied in, the final condensate polymer.

The particular average carbon number range which was most depended upon the ultimate environment of the alkyl phenol condensate which was produced thereby. That is, when used in connection with fuel oil formulations, additives made in accordance with the present invention will preferably utilize a slightly lower

average carbon number for these R groups.

More particularly, it was found that in connection with such fuel oils, including diesel fuels and heating oils, to maximize cloud point reduction an average carbon number of about $C_{18}$ was most desired, while to maximize pour point reduction an average carbon number of about $C_{16}$ was most desired.

On the other hand, in connection with lubricating oil compositions the average carbon number for maximizing pour point reduction was an average carbon content of from about $C_{18}$ to $C_{20}$.

Moreover, within each class of hydrocarbon oils, i.e. fuel or lubricating oil, each specific hydrocarbon oil can be associated with an optimum average carbon number for the R group (also referred to herein as the alkylate average carbon number) to achieve maximum cloud point or pour point depressancy relative to the base oil without any additive. Optimum pour depressancy will typically be achieved by an average carbon number that is lower than that needed to achieve optimum cloud pour point depressancy for a given hydrocarbon oil.

It was further found that while the molecular weight and molecular weight distribution ($M_w/M_n$) of the condensate polymer, degree of branching, and concentration of the condensation polymer in the hydrocarbon oil all affect, and are important for achieving low temperature flow performance, the two most dominant factors are the optimum alkylate average carbon number and the essential linearity of the alkyl group.

It was also believed that in any given situation the use of a range of alpha-olefins surrounding the optimum average carbon number was superior to the use of a single alpha-olefin having that number of carbon atoms. In any event, the most preferred alpha-olefins for use herein will thus include 1-hexadecene, 1-octadecene, 1-eicosene, 1-docosene, 1-tetracosene, and mixtures thereof.

A further important factor in conducting the alkylation reaction was the minimization of monofunctional alkylation product (e.g. most dialkylate products), and the maximization of difunctional alkylate products (e.g. mono alkylates) in the phenol alkylation reaction. As discussed hereinafter, the final alkyl phenol sulfur condensation product is synthesized to possess certain minimum requirements in terms of molecular weight and molecular weight distribution. If the alkylated phenol product mixture employed for condensation contains too much monofunctional dialkylate, then the final condensation polymer will not meet such requirements. This stems from the fact that when a second alkyl group attaches to the phenol to yield a 2,4- or 2,6-dialkyl phenol, it results in a monofunctional dialkylate molecule which, if reacted with a growing polymer chain, would terminate chain growth in the following manner:

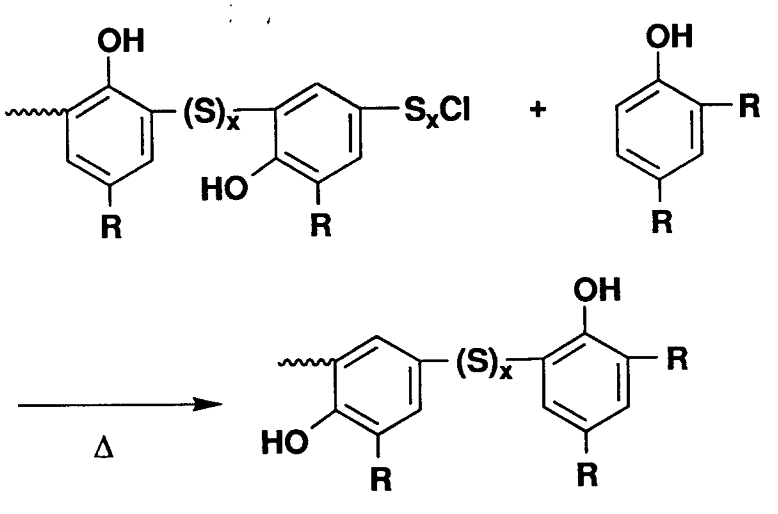

More specifically, the functionality of the alkylated-phenol reaction product expresses the number of available reactable sites, which remain on the alkylated phenol after alkylation, that can participate in the polymerization reaction through propagation of a growing polymer chain. The only freely reactable sites on an unsubstituted phenol molecule for purposes of polymerization are the 2-, 4-, and 6- carbons of the phenol aromatic ring. Thus, unsubstituted phenol is a trifunctional molecule. If monoalkylation occurs at only one of the 2-, 4-, or 6-positions, the resulting mono-alkylate is said to be difunctional, since one of the reactable sites has been eliminated through substitution of an alkyl group thereon. Similarly, the substitution of alkyl groups at any two of the 2-, 4-, or 6- carbons of the phenol molecule through dialkylation will result in the formation of a monofunctional dialkylate product. Thus, 2,4-dialkyl phenol and 2,6-dialkyl phenol are monofunctional dialkylates which will lead to chain termination, and thereby limit polymer molecular weights. While 2,5-dialkyl phenol and 3,5-dialkyl phenol are difunctional and trifunctional dialkyl monomers, respectively, such monomers do not normally form under typical alkylation conditions, because such formation would involve reaction at normally unreactive sites. Consequently, one seeks to minimize dialkylation generally, as most dialkylation leads to formation of monofunctional monomer. Thus, reference to dialkylation herein as being undesirable is technically a reference only to dialkylation which yields monofunctional dialkylate.

An equation relating the maximum degree of polymerization (DP) to the extent of reaction ($\rho$) and the functionality (f) of the reactants is referred to as the Modified Carothers Equation:

$$DP = 2/(2-\rho f)$$

This equation can be used to show that a monofunctional dialkylate monomer severely limits the maximum degree of polymerization in the alkyl phenol-sulfur condensation reaction.

As was disclosed in US-A-4976882 and 5039437, the use of separately synthesized tri- and tetrafunctional comonomers can be employed to increase the molecular weight of the final condensation polymer and/or to compensate for the presence of monofunctional dialkylate monomer.

Thus, the target molecular weight as disclosed was suitably achieved by controlling the amount of difunctional (e.g., monoalkylate) monomer to be typically at least about 80 mole %, and preferably at least about 85 mole %, and most preferably at least about 90 mole %, and typically from 80 to 100 mole %, preferably from 85 to 100 mole %, and most preferably from 90 to 100 (e.g., 95 to 100) mole %, based on the total moles of alkylate monomer in the monomer mixture intended for polymerization.

Correspondingly, the amount of monofunctional dialkylate monomer which can be tolerated will typically range from 0 to 20 mole %, preferably from 0 to 15 mole %, and most preferably from 0 to 10 (e.g. 0 to 5) mole % based on the moles of monomer in the alkylate monomer mixture.

High functionality monomers, such as the tri- and tetrafunctional comonomers described hereinafter, are typically employed in collective amounts of from 0 to 10 mole %, preferably from 2 to 8 mole %, and most preferably from 3 to 5 mole %, based on the total moles of alkylate monomer in the alkylate monomer mixture.

One way to minimize dialkylation in attempting to meet the condensation polymer molecular weight targets specified was to employ excess phenol relative to the olefin for the alkylation reaction. Accordingly, effective molar ratios of phenol to olefin can vary typically from 2:1 to 10:1 (or higher), preferably from 2:1 to 5:1. From a process standpoint, however, too much of an excess of phenol can be disadvantageous because of the need to remove the excess phenol from alkylation product after alkylation is completed.

Certain zeolite catalysts permit one to lower the phenol:olefin molar ratio to less than about 2:1, preferably between 1.7:1 and 1:1 and still achieve minimization of dialkylation. This low ratio extremely simplifies unreacted phenol recovery.

The alkylation reaction can generally be accomplished, within the above parameters, by a number of techniques known to those skilled in this art. One particularly suitable technique is described using the Friedel-Crafts reaction which occurs in the presence of a Lewis acid catalyst, such as boron trifluoride and its complexes with ethers, hydrogen fluoride, etc., aluminum chloride, aluminum bromide, and zinc dichloride, etc. Methods and conditions for carrying out such reactions are well known to those skilled in this art, and reference is made, for example, to the discussion in the article entitled "Alkylation of Phenols," in Kirk-Othmer Encyclopedia of Chemical Technology, 3rd Edition, Vol. 2, pp. 65-66, Interscience Publishers, Division of John Wiley and Company, New York, 1963, which is incorporated herein by reference thereto. A particularly preferred catalyst for use in such alkylation reactions is designated Amberlyst 15 by the Rohm and Haas Company. This catalyst is included among the strongly acidic macroreticular resins patented under US-A-4224415. This resin is itself composed of long chains of polystyrene locked together by divinylbenzene crosslinks into a three-dimensional, insoluble polymeric phase called a matrix, on which are attached sulfonic acid groups ($-SO_3H$). Amberlyst 15 possesses high acidity (4.7 meq/g), high porosity (32%) and high surface area (45 $m^2/g$).

In a highly preferred method for carrying out the alkylation reaction as disclosed herein, a zeolite catalyst is employed for use in the selective production of the desired mono-alkylate. More particularly, crystalline zeolites are used which have high silica to alumina ratios and which have effective pore sizes of between about 6 and 8 Angstroms, and include a number of commercial zeolite catalysts, such a LZ-Y82 catalyst manufactured by Union Carbide Corporation. In any event, a general description of these zeolites is set forth in US-A-4283573. In general, these zeolites have a crystal structure which provides access to and egress from the intracrystalline free space of the zeolites by virtue of having channels or networks of pores, the openings of which again preferably have a major dimension, or a free pore diameter, of between 6A and 8A. These zeolites are also characterized by pore apertures of about a size as would be provided by 12-member rings of silicon and aluminum atoms. The preferred types of zeolites for use in this invention possess a silica to alumina molar ratio of from 3:1 to 6:1. This ratio represents, as closely as possible, the ratio in the rigid anionic framework of the zeolite crystal. Furthermore, these preferred zeolites will have a high surface area, such as about 625 $m^2/g$. The use of these zeolite catalysts thus permits one to eliminate the expensive and difficult distillation step required to separate the mono-alkylate from the di-alkylate produced with the acid-type catalysts previously utilized.

In connection with the alkylated phenol product, the use of a linear alpha-olefin or a mixture of linear alpha-olefins gives a ratio of ortho to para attachments on the phenol of about 2:1. In contrast with the alkylated phenol product of this reaction, the use of a branched internal olefin or a mixture of branched internal olefins gives a ratio of ortho to para attachments on phenol of about 1:18. However, essentially linear alkyl groups attached either ortho or para to the hydroxy group perform equally well.

The next step in the preparation of the polymer additives using the essentially linear alkylated phenol prepared as disclosed herein is the actual polymerization or condensation reaction. The reaction itself is a condensation of the above-described alkyl phenol in the presence of a sulfurizing agent. The sulfurizing agent has been defined to be any compound or element which introduces $-(S)_x$-bridging groups between the alkylated phenol monomer groups, wherein x is a number of from 1 to about 8. Thus, the condensation reaction can be conducted with elemental sulfur or a halide thereof such as sulfur monochloride or, more preferably, sulfur dichloride. If elemental sulfur is used, this reaction is effected by heating the alkyl phenol compound at between 50 and 250 °C, and usually at least about 160 °C. The use of elemental sulfur typically yielded a mixture of bridging groups $-(S)_x-$ as described above. When sulfur halide was used, this reaction was effected by heating the alkyl phenol compound at between 50 and 120 °C, and usually at about 80 °C. Optimally, the reaction is conducted in the presence of a suitable diluent. The diluent can generally comprise a substantially inert organic diluent such as mineral oil or an alkane, ketone, ether, ether alcohol, or the like. In any event, the reaction is conducted for a period of time sufficient to effect substantial reaction. It is generally preferred to employ between 0.1 and 5 moles of the alkyl phenol material per equivalent of sulfurizing agent.

When elemental sulfur was used as the sulfurizing agent, it is frequently preferred to use a basic catalyst such as sodium hydroxide or an organic amine, preferably a heterocyclic amine (e.g., morpholine).

Particularly where sulfur halides are used as the sulfurizing agent, it is frequently preferred to use an acid acceptor, such as sodium hydroxide, calcium carbonate or the like, to react with the hydrogen halide evolved therein.

Pressure is not a critical factor, and can be atmospheric or below, up to 1000 psi or higher. Atmospheric pressure is preferred for convenience, and the pressure should be sufficient to maintain the reactants in the liquid phase.

The reactants, together with catalyst and any diluent which is employed, can thus be charged to a reactor and reacted under the conditions set forth above. The crude reaction product mixture can then be cooled, neutralized, water-washed to remove any catalyst, dried, and then stripped to remove excess reactant, any unreacted materials, and any diluent that may have been used.

The condensation reaction is conducted in a manner and under conditions sufficient to achieve or surpass certain minimum number average and weight average molecular weight targets. Accordingly, the condensation reaction is conducted to impart to the final polymer a number average molecular weight ($M_n$) as determined by vapor-phase osmometry of at least 3,000 (e.g., at least 4,000), preferably at least 5,000, and most preferably at least 7,000, and typically from 3,000 to 60,000 (e.g., 4,000 to 60,000), preferably from 5,000 to 30,000, most preferably from 7,000 to 20,000, and typically a weight average molecular weight ($M_w$) as determined by gel permeation chromatography, of at least 4,500 (e.g., at least 5,000), preferably at least 6,000, and typically from 4,500 to 100,000, preferably from 10,000 to 70,000 (e.g., 6,000 to 35,000), and most preferably from 20,000 to 50,000.

The maximum number and weight average molecular weights are limited only by the solubility of the condensate polymer in the particular hydrocarbon basestock in question.

These polymers have a ratio of weight average molecular weight to number average molecular weight ($M_w/M_n$), commonly referred to as molecular weight distribution, of greater than 1.5, preferably greater than 2.0, and most preferably greater than 2.5, and typically from 1.5 to 34, preferably from 2.0 to 24, and most preferably from 3.0 to 7.0. Generally, the higher the weight average molecular weight, the better suited or more effective these polymers are for improving the flow properties of various hydrocarbon oils in accordance with the present invention.

While number average molecular weight ($M_n$) can conveniently also be determined by gel permeation chromatography (GPC), it is considered that VPO techniques are more accurate, although the $M_n$ by the GPC technique will typically approximate $M_n$ by VPO within ± 1000, more typically ± 500.

In a preferred embodiment, polymers or condensates which are thus produced in accordance with the invention can be represented by the following formula:

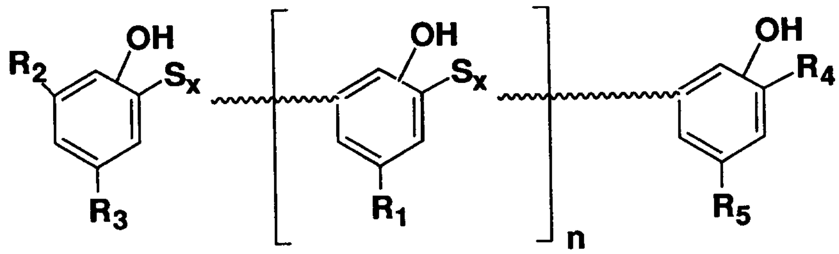

(I)

in which x is an integer of typically from 1 to about 8, preferably from 1 to 5, and most preferably from 1 to 2, $R_1$ represents attached essentially linear alkyl groups discussed above derived from the linear alpha-olefin having from 6 to 50 carbon atoms, in which the average number of carbon atoms in all of the groups constituting $R_1$ is between 12 and 26, preferably between 16 and 22, and most preferably between 18 and 20, and in which no more than 10 mole percent of alkyl groups have less than 12 carbon atoms and no more than 10 mole percent of alkyl groups have more than 26 carbon atoms; $R_2$, $R_3$, $R_4$ and $R_5$ independently can represent hydrogen or alkyl as described in connection with $R_1$, with the proviso that at least one of $R_2$ and $R_3$ is said alkyl and at least one of $R_4$ and $R_5$ is said alkyl. The hydroxy group of the phenol will be located on an aromatic carbon which is adjacent to a carbon on which at least one of the $-(S)_x-$ groups is attached.

The value of n is subject to the number average molecular weight targets discussed above, and the minimum value thereof expressed hereinafter will consequently vary depending on the average carbon number of the olefins employed for alkylation, the average value of x, and the number of repeating units controlled by n necessary to achieve such $M_n$ values when accounting for said olefin average carbon number and the average value of x.

Accordingly, n is a number which, subject to the above constraints, will typically be at least 3 (e.g., at least 5), preferably at least 8 (e.g., at least 10), and most preferably at least 12, and can vary typically from 5 to 80, preferably from 10 to 60, and most preferably from 15 to 30.

As indicated above, it can be somewhat difficult to increase the molecular weights of the alkylated phenol-sulfur condensates beyond a certain level because of the propensity of dialkylate monomers to terminate chain growth.

There are yet additional methods of increasing the molecular weight of the alkylated phenol-sulfur condensate flow improvers of the present invention. In this method, the polymerization step is carried out in the additional presence of trifunctional or tetrafunctional comonomer (functionality being reactable sites) so as to produce an ultimate condensation polymer having a branched backbone rather than linear backbone as shown in formula (I) hereabove wherein said linear backbones are crosslinked through said trifunctional and/or tetrafunctional comonomers.

In particular, a trifunctional comonomer having the following formula can be employed:

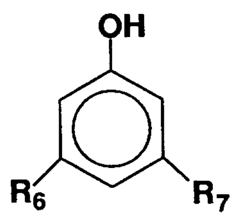

(II)

in which $R_6$ and $R_7$ can be hydrogen, alkyl, aryl, alkoxy, aryloxy, alkyl mercapto, and halogen. More particularly, it is preferred that $R_6$ and $R_7$ include branched or straight chain alkyl groups, preferably straight chain, such as $C_1$ through $C_{30}$ alkyl, preferably methyl, $C_6$ through $C_{14}$ aryl, $C_1$ through $C_{22}$ alkoxy, $C_6$ through $C_{14}$ aryloxy, $C_1$ to $C_{30}$ alkyl mercapto, and preferably halogens such as chlorine and bromine.

As discussed above, 3,5-dialkylate is difficult to achieve under normal alkylation conditions. Consequently, a variety of methods well known in the art can be employed to achieve 3,5-dialkylation. One such method involves a thallation reaction wherein, for example, 1,3-dimethyl benzene is contacted with a thallium trifluoro acetate catalyst to cause stereo specific oxidation to 3,5-dimethyl phenol.

Representative examples of trifunctional monomers include phenol, m-cresol, 3,5-xylenol, m-phenyl phenol, m-methoxyphenol, orcinol, and m-methyl mercapto phenol, while phenol is preferred.

For example, when phenol is employed as the trifunctional monomer, then a portion of the branched backbone can be represented by the following formula with an asterisk indicating the original phenol trifunctional monomer:

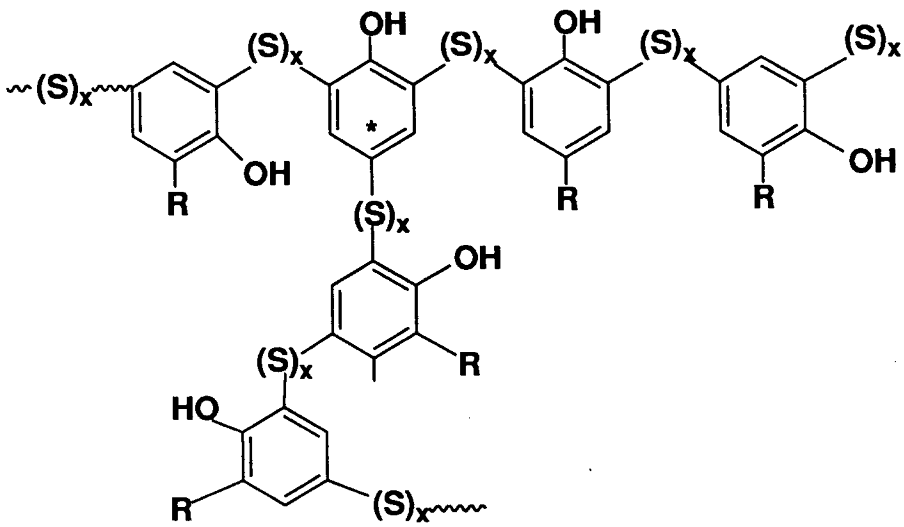

It is thus possible in this manner to produce such polymer condensates having weight average molecular weights determined by gel permeation chromatography of greater than about 10,000, preferably between 10,000 and 100,000, and most preferably greater than about 20,000.

Even further branching is achieved with tetrafunctional monomer which can crosslink four linear backbones.

The tetrafunctional comonomers which can be used in the polymerization step of the present invention can have the formula:

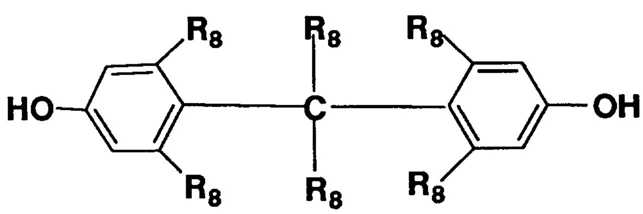

(III)

in which $R_8$ independently can be the same hydrogen, alkyl, aryl, alkoxy, aryloxy, alkyl mercapto, and halogen components discussed above in connection with the trifunctional comonomers as formula II hereof. Representative examples of suitable tetrafunctional monomers include bisphenol A, bisphenol B, methylene-4,4'-bis (3,5-dibutylphenol), methylene-4,4'-bis (3,5-dimethoxyphenol), methylene-4,4'-bis (3,5-dimethylmercapto phenol), with bisphenol A being preferred. Again, in this case it is also possible to produce such polymer condensates having weight average molecular weights determined by gel permeation chromatography of greater than about 10,000, preferably between 10,000 and 100,000, and most preferably greater than about 20,000.

The amount of such trifunctional and/or tetrafunctional comonomer employed in the polymerization or condensation step of the present invention must, however, be limited to a certain extent. That is, the amount of comonomer present should be less than 10 wt. % of a combination of the alkylated phenol and the

sulfurizing agent, and preferably less than 8 wt. %. It has thus been found that if too great an amount of the trifunctional and/or tetrafunctional comonomer is present, that material tends to crosslink to the extent that an insoluble mass can be formed thereby. This can be avoided, however, by using the amounts discussed above, and additionally by conducting the polymerization in the initial presence of small amounts of the trifunctional or tetrafunctional comonomer. Also, this comonomer can be continuously added during the course of polymerization, thereby becoming diluted with the polymerizing alkyl phenol composition to maintain the comonomer as dilute as possible throughout the polymerization reaction.

It is also contemplated, although less preferred, that blends of separately synthesized alkyl phenol condensates meeting the aforedescribed requirements can be employed.

For purpose of discussion, when such blends are employed, the overall alkylate average carbon number for each polymer component in the blend in which the alkylate portion thereof is derived from a single alpha-olefin, or single mixture of alpha-olefins, can also be referred to herein as the alkylate intra-molecular carbon average. However, the alkylate intra-molecular carbon average of each polymer component in the blend can then also be averaged on a molar basis to determine what is referred to herein as the alkylate inter-molecular carbon average for the blend.

It is believed that when the optimum alkylate average carbon number (i.e., intra-molecular average carbon number) has been determined for a particular hydrocarbon oil, the best low temperature performance is achieved by a single polymer which possesses this optimum average carbon number value, rather than a blend of polymers wherein each polymer component in the blend possesses a non-optimum alkylate intra-molecular carbon average, but the blend collectively possesses an alkylate inter-molecular carbon average value equal to the value of the optimum intra-molecular carbon average.

The polymer additives produced in accordance with the present invention have been found to be useful in fuel oils and lubricating oils. The normally liquid fuel oils are generally derived from petroleum sources, e.g., normally liquid petroleum distillate fuels, though they may include those produced synthetically by the Fischer-Tropsch and related processes, the processing of organic waste material or the processing of coal, lignite or shale rock.

The additives derived from the process of this invention find their primary utility, however, in lubricating oil compositions, which employ a base oil in which the additives are dissolved or dispersed. Such base oils may be natural or a mixture of natural and synthetic oils.

Thus, base oils suitable for use in preparing the lubricating oil compositions of the present invention include those conventionally employed as crankcase lubricating oils for spark-ignited and compression-ignited internal combustion engines, such as automobile and truck engines, marine and railroad diesel engines, and the like. Advantageous results are also achieved by employing the additives derived from the process of the present invention in base oils conventionally employed in and/or adapted for use as power transmitting fluids such as automatic transmission fluids, tractor fluids, universal tractor fluids and hydraulic fluids, heavy duty hydraulic fluids, power steering fluids and the like. Gear lubricants, industrial oils, pump oils and other lubricating oil compositions can also benefit from the incorporation therein of the additives derived from the process of the present invention.

Thus, the additives produced as disclosed herein may be suitably incorporated into mixtures of natural and synthetic base oils provided these mixtures include at least about 80 wt. % of natural base oil. Suitable synthetic base oils for use in these mixtures include alkyl esters of dicarboxylic acids, polyglycols and alcohols; polyalpha-olefins, polybutenes, alkyl benzenes, organic esters of phosphoric acids, polysilicone oils, etc.

The lubricating oil base stock conveniently has a viscosity of typically 2.5 to 12, and preferably 2.5 to 9 cSt at 100 °C.

Thus, the additives of the present invention can be employed in a hydrocarbon oil (i.e., fuel oil or lubricating oil) composition which comprises hydrocarbon oil, typically in a major amount, and the additive, typically in a minor amount, which is effective to impart or enhance one or more of the low temperature flow properties described herein. Additional conventional additives selected to meet the particular requirements of a selected type of hydrocarbon oil composition can be included as desired.

The additives of this invention are oil-soluble, dissolvable in oil with the aid of a suitable solvent, or are stably dispersible materials. Oil-soluble, dissolvable, or stably dispersible as that terminology is used herein does not necessarily indicate that the materials are soluble, dissolvable, miscible, or capable of being suspended in oil in all proportions. It does mean, however, that the additives, for instance, are soluble or stably dispersible in oil to an extent sufficient to exert their intended effect in the environment in which the oil is employed. Moreover, the additional incorporation of other additives may also permit incorporation of higher levels of a particular polymer adduct hereof, if desired.

Accordingly, while any effective amount of these additives can be incorporated into the fully formulated hydrocarbon oil composition, it is contemplated that such effective amount be sufficient to provide said hydrocarbon oil composition with an amount of the additive of typically from 0.005 to 10, e.g., 0.01 to 2, and preferably from 0.025 to 0.25 wt. %, based on the weight of said composition.

The additives of the present invention can be incorporated into the hydrocarbon oil in any convenient way. Thus, they can be added directly to the oil by dispersing, or dissolving the same in the oil at the desired level of concentration, typically with the aid of a suitable solvent such as toluene, cyclohexane, or tetrahydrofuran. Such blending can occur at room temperature or elevated temperatures. In this form the additive per se is thus being utilized as a 100% active ingredient form which can be added to the oil or fuel formulation by the purchaser. Alternatively, these additives may be blended with a suitable oil-soluble solvent and/or base oil to form a concentrate, which may then be blended with a hydrocarbon oil base stock to obtain the final formulation. Concentrates will typically contain from about 1 to 50%, by weight of the additive, and preferably from about 10 to 30% by weight of the additive.

The hydrocarbon oil base stock for the additives prepared as disclosed in this invention typically is adapted to perform a selected function by the incorporation of additives therein to form lubricating oil compositions (i.e., formulations).

Representative additives typically present in such formulations include viscosity modifiers, corrosion inhibitors, oxidation inhibitors, friction modifiers, dispersants, anti-foaming agents, anti-wear agents, pour point depressants, detergents, rust inhibitors and the like.

Representative examples of such materials, and their methods of preparation, are found in EP-A-208560.

Compositions when containing these conventional additives are typically blended into the base oil in amounts which are effective to provide their normal attendant function. Representative effective amounts of such additives are illustrated as follows:

| Additive | Wt. % a.i. (Broad) | Wt. % a.i. (Preferred) |
|---|---|---|
| Viscosity Modifier | 0.01-12 | 0.01-4 |
| Corrosion Inhibitor | 0.01-5 | 0.01-1.5 |
| Oxidation Inhibitor | 0.01-5 | 0.01-1.5 |
| Dispersant | 0.1-20 | 0.1-8 |
| Pour Point Depressant | 0.005-10 | 0.01-2 |
| Anti-Foaming Agents | 0.001-3 | 0.001-0.15 |
| Anti-Wear Agents | 0.001-5 | 0.001-1.5 |
| Friction Modifiers | 0.01-5 | 0.01-1.5 |
| Detergents/Rust Inhibitors | 0.01-10 | 0.01-3 |
| Mineral Oil Base | Balance | Balance |

When other additives are employed, it may be desirable, although not necessary, to prepare additive concentrates comprising concentrated solutions or dispersions of the flow improver (in concentrate amounts hereinabove described), together with one or more of said other additives (said concentrate when constituting an additive mixture being referred to herein as an additive-package) whereby several additives can be added simultaneously to the base oil to form the hydrocarbon oil composition. Dissolution of the additive concentrate into the hydrocarbon oil may be facilitated by solvents and by mixing accompanied with mild heating, but this is not essential. The concentrate or additive-package will typically be formulated to contain the flow improver additive and optional additional additives in proper amounts to provide the desired concentration in the final formulation when the additive-package is combined with a predetermined amount of base hydrocarbon oil. Thus, the product produced by the process of the present invention can be added to small amounts of base oil or other compatible solvents along with other desirable additives to form additive-packages containing active ingredients in collective amounts of typically from about 2.5 to about 90%, and preferably from about 5 to about 75%, and most preferably from about 8 to about 50% by weight additives in the appropriate proportions with the remainder being base oil. For safety considerations, the base oil for concentrates is typically a lubricating oil rather than a fuel oil.

The final formulations may employ typically about 10 wt. % of the additive-package with the remainder being base oil.

All of said weight percents expressed herein are based on active ingredient (a.i.) content of the additive, and/or upon the total weight of any additive-package, or formulation which will be the sum of the a.i. weight of each additive plus the weight of total oil or diluent.

This invention will be further understood by reference to the following examples, wherein all parts are parts by weight and all molecular weights are either number average molecular weight determined by vapor-phase osmometry or weight average molecular weights determined by gel permeation chromatography as noted unless otherwise specified, and which include preferred embodiments of the invention.

The following examples are given as specific illustrations of the claimed invention. It should be understood, however, that the invention is not limited to the specific details set forth in the examples. All parts and percentages in the examples, as well as in the remainder of the specification, are by weight unless otherwise specified.

**EXAMPLE 1 (Comparative)**

This example is directed to the preparation of a typical alkylated phenol component using the process disclosed in US-A-4976882 and alkyl phenol-sulfur condensates produced thereby. Octadecyl phenol was prepared by charging into a four-neck, 5-liter round bottom flask equipped with a mechanical stirrer, 933 grams of phenol (9.93 moles) and 286 grams of Amberlyst 15 catalyst. A reflux condenser, a thermometer, an addition funnel, and a nitrogen inlet tube were attached to the flask and the mixture was heated to 70 °C. With stirring under a blanket of nitrogen, 834 grams (3.31 moles) of 1-octadecene was added dropwise over a period of about one hour. The temperature was raised to 90 °C and maintained at this temperature for four hours. The reaction mixture was then cooled to 50 °C and filtered to remove the catalyst. The excess phenol was removed by vacuum distillation. The yield was 1,008 grams or 88%. The product has a refractive index of 1.4859 at 25 °C, a viscosity of 38.0 cP at 40 °C, and a hydroxyl number of 144 mg KOH/g. The infrared spectrum of the product showed absorption bands at 830 and 750 $cm^{-1}$m which are characteristic of alkyl phenols. The aromatic substitution pattern was determined by $^{13}C$-NMR spectroscopy and showed that the ortho to para ratio was 2.0:1.0. The alkyl substitution pattern was determined by $^{1}H$-NMR spectroscopy and showed that the product consisted of 50 mole % 2-substituted alkylate and 50 mole % ≧ 3-substituted alkylate.

**EXAMPLE 2 (Comparative)**

In order to demonstrate the criticality of the linearity of the alkyl group used in the alkyl phenol-formaldehyde condensates, Example 1 was repeated, except that in this case the mixture was heated to 115 °C instead of 90 °C. The yield of octadecyl phenol was 893 grams, or 78%. The product had a hydroxyl number of 138 mg KOH/g, and its infrared spectrum showed absorptions at 830 and 750 $cm^{-1}$, which are characteristic of alkyl phenols. The aromatic substitution pattern was determined by $^{13}C$-NMR spectroscopy and showed that the ortho to para ratio was 1.8:1.0. The alkyl substitution pattern was determined by the $^{1}H$-NMR spectroscopy and showed that the product consisted of 35 mole % 2-substituted alkylate and 65 mole % ≧ 3-alkylate. The greater degree of rearrangement in this alkyl phenol was due to the higher reaction temperature.

**EXAMPLE 3**

In order to demonstrate the method used in the present invention to produce alkyl phenols with a less rearrangement, Example 1 was repeated, except that a dipolar aprotic cosolvent, nitrobenzene, was added to the reaction mixture. Into a four-neck 1-liter round-bottom flask equipped with a mechanical stirrer, 125 grams of phenol (1.33 moles), 31.5 grams of Amberlyst 15 catalyst, and 164 grams of nitrobenzene were charged. A reflux condenser, a thermometer, an addition funnel and a nitrogen inlet tube were attached to the flask and the mixture was heated to 70 °C. With stirring under a blanket of nitrogen, 109 grams (0.43 moles) of 1-octadecene was added dropwise over a period of about one hour. The temperature was raised to 90 °C and maintained at this temperature for four hours. The reaction mixture was then cooled to 50 °C and filtered to remove the catalyst. The excess phenol and nitrobenzene were removed by vacuum distillation. The yield was 207 grams, or 99%. The infrared spectrum of the product showed absorption bands at 830 and 750 $cm^{-1}$, which are characteristic of alkyl phenols. The aromatic substitution pattern was determined by $^{13}C$-NMR spectroscopy and showed that the ortho to para ratio was 1.7:1.0. The alkyl substitution pattern was determined by $^{1}H$-NMR spectroscopy and showed that the product consisted of 59 mole % 2-substituted alkylate and 41 mole % ≧ 3-substituted alkylate, i.e., 18% less rearrangement than the alkylate produced in accordance with Example 1.

**EXAMPLE 4 (Comparative)**

This is an example of the preparation of an alkylated phenol-sulfur condensate polymer from a conventional alkylated phenol. Into a four-necked, 1 liter round bottom flask, equipped with a mechanical stirrer, a thermometer, addition funnel, nitrogen inlet tube, and a Dean-Stark trap with a reflux condenser, were charged 250 grams of octadecyl phenol produced in accordance with Example 1. The octadecyl phenol was heated to 80 °C. While stirring, 82 grams of a mixture of 76% sulfur dichloride and 24% sulfur monochloride were added over a period of about one hour, with the temperature maintained at about 80 °C. After the addition was completed, the reaction mixture was soaked for about 5 minutes at 80 °C and then heated to 95 °C and sparged with nitrogen for one hour. While continuing stirring, 107 grams of diluent oil were added, and the mixture was stirred for 15 minutes and cooled to room temperature. The product had a sulfur content of 7.50%. The number average molecular weight (VPO) of this polymer was 4,900, and its weight average molecular weight (GPC) was 11,000.

**EXAMPLE 5 (Comparative)**

As another example of the preparation of an alkyl phenol-sulfur condensate polymer, Example 4 was repeated, except that the octadecyl phenol produced in accordance with Example 2 was used. The sulfur content of the product was 7.42%. The number average and weight average molecular weights of the dialyzed polymer by gel permeation chromatography were 4,400 and 10,100, respectively.

**EXAMPLE 6**

This is another example of the preparation of an alkyl phenol-sulfur condensate polymer according to the invention. Example 4 was repeated, except that the octadecyl phenol produced in accordance with Example 3 was used. The sulfur content of the product was 7.48%. The number average and weight average molecular weights of the polymer were 4,800 and 9,300, respectively.

**EXAMPLE 7**

In order to demonstrate the criticality of the linearity of the alkyl groups used in the alkyl phenol-sulfur condensates, the octadecyl phenol-sulfur condensates produced in accordance with Examples 4-6 were tested for pour point depressancy in a lube base stock (Exxon S150N). Pour points were measured according to ASTM D 97 method, and the results are set forth in Table 1 below. These results demonstrate that: (1) the 15-mole % reduction in alkylate containing a pendant method group on the 2-carbon and a corresponding increase in alkylate having substitution on the 3- or higher carbon decreases pour point depressancy significantly (compare runs 1, 2 and 5); (2) the 9-mole % increase in alkylate containing a pendant method group on the 2-carbon and a corresponding decrease in the alkylate having substitution on the 3- or higher carbon increased pour point depressancy significantly (compare runs 1, 3 and 9).

The decreased pour point depressancy of the octadecyl phenol-sulfur condensate produced in accordance with Example 5, compared with Example 4, is because of the greater degree of rearrangement in the alkylate used, i.e., Example 2. This results from a higher reaction temperature.

The increased pour point depressancy of the octadecyl phenol-sulfur condensate produced in accordance with Example 6, compared to Example 4, is because of less rearrangement in the alkylate used, i.e., Example 3. This results from the use of the dipolar aprotic cosolvent, nitrobenzene.

Table 1

| Pour Point Depressancy of Octadecyl Phenol-Sulfur Condensates with Varying Degrees of Alkylate Rearrangement | | | | |
|---|---|---|---|---|
| **Run** | **Additive** | **Additive Conc. (Wt %)** | **2-Substituted Alkylate (5)** | **Pour Point (°F)** |
| 1 | Nil | 0.00 | -- | +10, +15, +15 |
| 2 | Example 4 | 0.05 | 50 | -15, -15, -10 |
| 3 | Example 4 | 0.10 | 50 | -25, -25, -20 |
| 4 | Example 4 | 0.20 | 50 | -40, -40, -35 |
| 5 | Example 5 | 0.05 | 35 | +10, +10, +15 |
| 6 | Example 5 | 0.10 | 35 | -15, -15, -10 |
| 7 | Example 5 | 0.20 | 35 | -20, -20, -20 |
| 8 | Example 6 | 0.05 | 59 | -20, -20, -15 |
| 9 | Example 6 | 0.10 | 59 | -40, -40, -35 |
| 10 | Example 6 | 0.20 | 59 | -40, -40, -40 |

## Claims

**1.** A method of producing such sulfur bridged alkyl phenol condensates capable of improving the low temperature flow properties and oxidation stability of hydrocarbon oils wherein the condensation reaction product is prepared by a method which comprises a method for producing a polymeric additive suitable for improving the low temperature flow properties and oxidative stability of hydrocarbon oil which comprises (1) preparing an alkylated phenol by the reaction in a dipolar aprotic cosolvent of (a) phenol and (b) linear alpha-olefin having (i) from 6 to 50 carbon atoms, (ii) an average carbon number of from 12 to 26; and (iii) not more than 10 mole % containing less than 12 carbon atoms and not more than 10 mole % containing more than 26 carbon atoms to form an alkylated phenol comprising at least 80 mole % difunctional alkylated phenol and having greater than 35 mole % of the alkyl groups derived from the linear alpha-olefin as alpha methyl-substituted linear alkyl groups; and (2) condensing said alkylated phenol of step (1) with a sulfurizing agent so as to produce a sulfur bridged condensate having a number average molecular weight of at least 3,000 (as measured by VPO) and molecular weight distribution of at least about 1.5, the bridge comprising a group of the formula $-(S)_x-$ where x is a number between 1 and 8.

**2.** The method of claim 1 wherein said sulfurizing agent is elemental sulfur or a sulfur-containing compound having the formula $S_xCl_2$, wherein x is an integer from 1 to 2.

**3.** The method of claim 1 or claim 2 wherein said condensing step is conducted in the further presence of at least one comonomer represented of either the formula:

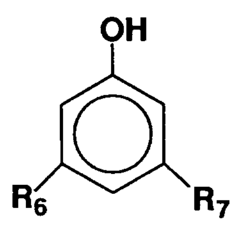

(II)

or the formula:

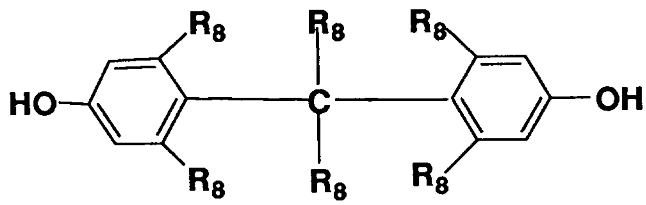

(III)

wherein $R_6$, $R_7$ and $R_8$ are each independently selected from the group consisting of hydrogen, alkyl, aryl, alkoxy, aryloxy, alkyl mercapto, and halogen,
and wherein said comonomer is present in an amount of less than 10 wt. % of the combination of alkylated phenol and sulfurizing agent.

**4.** The method of any of claims 1 to 3 wherein the dipolar-aprotic cosolvent has a dielectric constant of greater than 10.

**5.** The method of claim 4 wherein the dipolar-aprotic solvent is nitrobenzene; nitromethane; N,N-dimethylformamide; acetonitrile, sulfolane or dimethyl sulfoxide.

**6.** The method of any of claims 1 to 5 wherein said alpha-olefins have average carbon atom numbers of from 18 to 20.

**7.** The method of any of claims 1 to 6 wherein the alkylation is conducted at a temperature of from 50 ° C to 200 ° C.

**8.** The method of claim 7 wherein the alkylation is conducted at or below 100 ° C.

**9.** The method of any of claims 1 to 8 wherein the molar ratio of phenol to olefin ranges from 2:1 to 10:1.

**10.** The method of any of claims 1 to 8 wherein said alkylation is conducted in the presence of an acidic crystalline aluminasilicate zeolite catalyst in order to minimize the production of dialkylate therein.

**11.** The method of claim 10 wherein the zeolite catalyst has a silica to alumina molar ratio of from 3:1 to 6:1.

**12.** The method of claim 11 wherein said zeolite catalyst has a free pore diameter of between 6A and 8A.

**13.** The method of any of claims 10 to 12 wherein said alkylation is conducted using a molar ratio of said phenol to said linear alpha-olefin of less than 2:1.

**14.** The method of claim 13 wherein said alkylation is conducted using a molar ratio of said phenol to said linear alpha-olefin of between 1.7:1 and 1:1.

**15.** The use of an alkylated phenol comprising at least 80 mole % difunctional alkylated phenol and having greater than 35 mole % of the alkyl groups derived from the linear alpha-olefin as alpha methyl-substituted linear alkyl groups prepared by the reaction in a dipolar aprotic cosolvent of (a) phenol and (b) linear alpha-olefin having (i) from 6 to 50 carbon atoms, (ii) an average carbon number of from 12 to 26; and (iii) not more than 10 mole % containing less than 12 carbon atoms and not more than 10 mole % containing more than 26 carbon atoms to form a sulfur bridged condensate having a number average molecular weight of at least 3,000 (as measured by VPO) and molecular weight distribution of at least about 1.5, the bridge comprising a group of the formula $-(S)_x-$ where x is a number between 1 and 8.

## Patentansprüche

1. Verfahren zur Herstellung von solchen schwefelverbrückten Alkylphenolkondensaten, die zur Verbesserung der Tieftemperaturfließeigenschaften und der Oxidationsbeständigkeit von Kohlenwasserstoffölen in der Lage sind, wobei das Kondensationsreaktionsprodukt nach einem Verfahren hergestellt ist, das ein Verfahren zur Herstellung eines zur Verbesserung der Tieftemperaturfließeigenschaften und der Oxidationsbeständigkeit von Kohlenwasserstofföl geeigneten polymeren Additivs umfaßt, bei dem (1) ein alkyliertes Phenol hergestellt wird, indem (a) Phenol und (b) lineares $\alpha$-Olefin mit (i) 6 bis 50 Kohlenstoffatomen, (ii) einer durchschnittlichen Kohlenstoffzahl von 12 bis 26 und (iii) nicht mehr als 10 Mol.%, die weniger als 12 Kohlenstoffatome enthalten, und nicht mehr als 10 Mol.%, die mehr als 26 Kohlenstoffatome enthalten, in einem dipolar-aprotischen Colösungsmittel unter Bildung eines alkylierten Phenols umgesetzt werden, das mindestens 80 Mol.% difunktional alkyliertes Phenol umfaßt und bei dem mehr als 35 Mol.% der von den linearen $\alpha$-Olefinen abgeleiteten Alkylgruppen als $\alpha$-methylsubstituierte lineare Alkylgruppen vorliegen, und (2) das alkylierte Phenol aus Stufe (1) mit einem Sulfurierungsmittel kondensiert wird, um so ein schwefelverbrücktes Kondensat mit einem durchschnittlichen Molekulargewicht (Zahlenmittel) von mindestens 3000 (gemessen mittels VPO) und einer Molekulargewichtsverteilung von mindestens etwa 1,5 herzustellen, wobei die Brücke eine Gruppe mit der Formel $-(S)_x-$ umfaßt, in der x eine Zahl zwischen 1 und 8 ist.

2. Verfahren nach Anspruch 1, bei dem das Sulfurierungsmittel elementarer Schwefel oder eine schwefelhaltige Verbindung mit der Formel $S_xCl_2$ ist, wobei x eine ganze Zahl von 1 bis 2 ist.

3. Verfahren nach Anspruch 1 oder Anspruch 2, bei dem die Kondensationsstufe außerdem in Gegenwart von mindestens einem Comonomer durchgeführt wird, das entweder durch die Formel

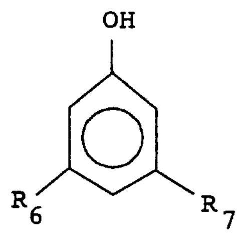

(II)

oder durch die Formel

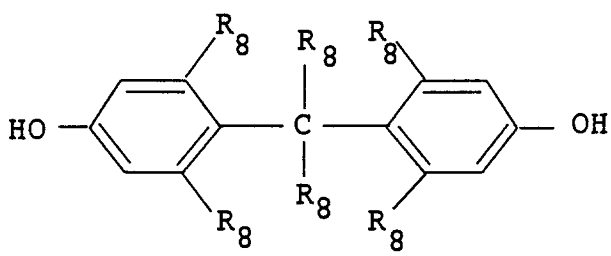

(III)

wiedergegeben wird, wobei $R_6$, $R_7$ und $R_8$ jeweils unabhängig ausgewählt sind aus der Gruppe bestehend aus Wasserstoff, Alkyl, Aryl, Alkoxy, Aryloxy, Alkylmercapto und Halogen, und bei dem das Comonomer in einer Menge von weniger als 10 Gew.% der Kombination aus alkyliertem Phenol und Sulfurierungsmittel vorhanden ist.

4. Verfahren nach einem der Ansprüche 1 bis 3, bei dem das dipolar-aprotische Colösungsmittel eine Dielektrizitätskonstante von mehr als 10 aufweist.

5. Verfahren nach Anspruch 4, bei dem das dipolar-aprotische Colösungsmittel Nitrobenzol, Nitromethan, N,N-Dimethylformamid, Acetonitril, Sulfolan oder Dimethylsulfoxid ist.

6. Verfahren nach einem der Ansprüche 1 bis 5, bei dem die α-Olefine durchschnittliche Kohlenstoffatomzahlen von 18 bis 20 aufweisen.

7. Verfahren nach einem der Ansprüche 1 bis 6, bei dem die Alkylierung bei einer Temperatur von 50 °C bis 200 °C durchgeführt wird.

8. Verfahren nach Anspruch 7, bei dem die Alkylierung bei 100 °C oder unter 100 °C durchgeführt wird.

9. Verfahren nach einem der Ansprüche 1 bis 8, bei dem das Molverhältnis von Phenol zu Olefin im Bereich von 2:1 bis 10:1 liegt.

10. Verfahren nach einem der Ansprüche 1 bis 8, bei dem die Alkylierung in Gegenwart eines sauren kristallinen Aluminiumoxid-Silikat-Zeolithkatalysators durchgeführt wird, um deren Produktion von Dialkylat zu minimieren.

11. Verfahren nach Anspruch 10, bei dem der Zeolithkatalysator ein Molverhältnis von Siliciumdioxid zu Aluminiumoxid von 3:1 bis 6:1 aufweist.

12. Verfahren nach Anspruch 11, bei dem der Zeolithkatalysator einen freien Porendurchmesser zwischen 6 Å und 8 Å aufweist.

13. Verfahren nach einem der Ansprüche 10 bis 12, bei dem die Alkylierung unter Verwendung eines Molverhältnisses von dem Phenol zu dem linearen α-Olefin von weniger als 2:1 durchgeführt wird.

14. Verfahren nach Anspruch 13, bei dem die Alkylierung unter Verwendung eines Molverhältnisses von dem Phenol zu dem linearen α-Olefin zwischen 1,7:1 und 1:1 durchgeführt wird.

15. Verwendung eines alkylierten Phenols, das mindestens 80 Mol.% difunktional alkyliertes Phenol umfaßt und bei dem mehr als 35 Mol.% der von linearen α-Olefinen abgeleiteten Alkylgruppen als α-methylsubstituierte lineare Alkylgruppen vorliegen, und das durch die Umsetzung von (a) Phenol und (b) linearem α-Olefin mit (i) 6 bis 50 Kohlenstoffatomen, (ii) einer durchschnittlichen Kohlenstoffzahl von 12 bis 26 und (iii) nicht mehr als 10 Mol.%, die weniger als 12 Kohlenstoffatome enthalten, und nicht mehr als 10 Mol.%, die mehr als 26 Kohlenstoffatome enthalten, in einem dipolaraprotischen Colösungsmittel hergestellt worden ist, zur Bildung eines schwefelverbrückten Kondensats mit einem durchschnittlichen Molekulargewicht (Zahlenmittel) von mindestens 3000 (gemessen mittels VPO) und einer Molekulargewichtsverteilung von mindestens etwa 1,5, wobei die Brücke eine Gruppe mit der Formel $-(S)_x-$ umfaßt, in der x eine Zahl zwischen 1 und 8 ist.

## Revendications

1. Procédé de préparation de produits de condensation d'alkylphénols pontés par du soufre, capables d'améliorer les propriétés d'écoulement à basse température et la stabilité à l'oxydation d'huiles hydrocarbonées, dans lequel le produit de réaction de condensation est préparé par un procédé qui comprend un procédé de production d'un additif polymérique convenant pour améliorer les propriétés d'écoulement à basse température et la stabilité à l'oxydation d'une huile hydrocarbonée, qui comprend (1) la préparation d'un phénol alkylé par la réaction d'un cosolvant aprotique dipolaire (a) de phénol et (b) d'une alpha-oléfine linéaire ayant (i) 6 à 50 atomes de carbone, (ii) un nombre moyen d'atomes de carbone de 12 à 26 ; et (iii) un maximum de 10 moles % contenant moins de 12 atomes de carbone et un maximum de 10 moles % contenant plus de 26 atomes de carbone pour former un phénol alkylé comprenant au moins 80 moles % de phénol alkylé difonctionnel et portant plus de 35 moles % de groupes alkyle dérivés de l'alpha-oléfine linéaire comme groupes alkyle linéaires à substitution alpha-méthyle ; et (2) la condensation du phénol alkylé de l'étape (1) avec un agent de sulfuration afin d'obtenir un produit de condensation ponté par du soufre ayant une moyenne numérique de poids moléculaire d'au moins 3000 (comme mesuré par osmométrie en phase vapeur) et une distribution de poids moléculaire d'au moins environ 1,5, le pont comprenant un groupe de formule $-(S)_x-$ dans laquelle $\underline{x}$ est un nombre compris entre 1 et 8.

2. Procédé suivant la revendication 1, dans lequel l'agent de sulfuration est le soufre élémentaire ou un composé contenant du soufre répondant à la formule $S_xCl_2$ dans laquelle x est un nombre entier égal à 1 ou 2.

3. Procédé suivant la revendication 1 ou la revendication 2, dans lequel l'étape de condensation est conduite en la présence supplémentaire d'au moins un comonomère représenté par la formule :

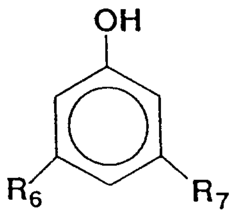

(II)

ou par la formule :

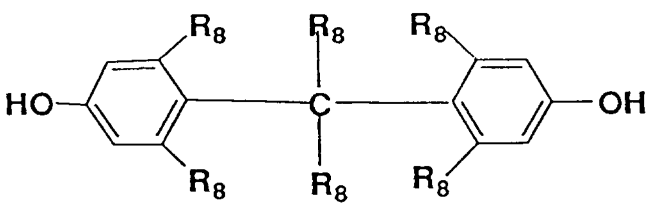

(III)

formules dans lesquelles $R_6$, $R_7$ et $R_8$ sont choisis chacun, indépendamment, dans le groupe comprenant l'hydrogène, un radical alkyle, aryle, alkoxy, aryloxy, alkylmercapto et un halogène,

et le comonomère est présent en une quantité inférieure à 10 % en poids de l'association phénol alkylé plus agent de sulfuration.

4. Procédé suivant l'une quelconque des revendications 1 à 3, dans lequel le cosolvant aprotique dipolaire a une constante diélectrique supérieure à 10.

5. Procédé suivant la revendication 4, dans lequel le solvant aprotique dipolaire est le nitrobenzène ; le nitrométhane ; le N,N-diméthylformamide ; l'acétonitrile, le sulfolane ou le diméthylsulfoxyde.

6. Procédé suivant l'une quelconque des revendications 1 à 5, dans lequel les alpha-oléfines en question ont des nombres moyens d'atomes de carbone de 18 à 20.

7. Procédé suivant l'une quelconque des revendications 1 à 6, dans lequel l'alkylation est conduite à une température de 50 à 200 ° C.

8. Procédé suivant la revendication 7, dans lequel l'alkylation est conduite à une température égale ou inférieure à 100 ° C.

9. Procédé suivant l'une quelconque des revendications 1 à 8, dans lequel le rapport molaire du phénol à l'oléfine se situe dans la plage de 2:1 à 10:1.

10. Procédé suivant l'une quelconque des revendications 1 à 8, dans lequel l'alkylation est conduite en présence d'un catalyseur cristallin acide formé d'une zéolite du type aluminosilicate afin d'y minimiser la formation d'un produit de dialkylation.

11. Procédé suivant la revendication 10, dans lequel le catalyseur zéolitique a un rapport molaire silice:alumine de 3:1 à 6:1.

12. Procédé suivant la revendication 11, dans lequel le catalyseur zéolitique a un diamètre libre des pores compris entre 6 et 8 Å.

13. Procédé suivant l'une quelconque des revendications 10 à 12, dans lequel on conduit l'alkylation en utilisant un rapport molaire du phénol à l'alpha-oléfine linéaire inférieur à 2:1.

14. Procédé suivant la revendication 13, dans lequel on conduit l'alkylation en utilisant un rapport molaire du phénol à l'alpha-oléfine linéaire compris entre 1,7:1 et 1:1.

15. Utilisation d'un phénol alkylé comprenant au moins 80 moles % de phénol alkylé difonctionnel et dont plus de 35 moles % des groupes alkyle sont dérivées de l'alpha-oléfine linéaire comme groupes alkyle linéaires à substituant méthyle en alpha, préparé par la réaction, dans un cosolvant aprotique dipolaire, (a) de phénol et (b) d'une alpha-oléfine linéaire ayant (i) 6 à 50 atomes de carbone, (ii) un nombre moyen d'atomes de carbone de 12 à 26 ; et (iii) un maximum de 10 modes % contenant moins de 12 atomes de carbone et un maximum de 10 moles % contenant plus de 26 atomes de carbone, pour former un produit de condensation ponté par du soufre ayant une moyenne numérique de poids moléculaire d'au moins 3000 (comme mesuré par osmométrie en phase vapeur) et une distribution de poids moléculaire d'au moins environ 1,5, le pont comprenant un groupe de formule $-(S)_x-$ dans laquelle x est un nombre de valeur comprise entre 1 et 8.